# EUROPEAN PATENT APPLICATION

(11) **EP 4 018 937 A1**
(43) Date of publication of application: **29.06.2022**
(21) Application number: 19942043.1
(22) Date of filing: 17.09.2019
(51) Int. Cl.: A61B 10/02

(54) **BONE MARROW HARVESTING DEVICE**

(30) Priority: 21.08.2019 KR 20190102121
(71) Applicant: Rev-Med, Inc., Seongnam-si, Gyeonggi-do 13229 (KR)
(72) Inventor: SHIN, Bong Geun, Suwon-si, Gyeonggi-do 16517 (KR)
(74) Representative: Grant, David Michael
(86) International application number: PCT/KR2019/011987
(87) International publication number: WO 2021/033826

(57) **Abstract**

The present invention relates to a bone marrow harvesting device. More specifically, the present invention relates to a bone marrow harvesting device which, when harvesting bone marrow, can not only easily and stably move the position of a catheter inserted into a bone marrow cavity, but can also harvest the bone marrow in a state where the catheter is stably fixed after the position of the catheter is moved. To this end, the bone marrow harvesting device comprises: a hub inserted into a bone marrow cavity; a catheter inserted into the hub and having a syringe detachably connected to one side thereof; and a holder connected to one end of the catheter such that the catheter is movable along the longitudinal direction.

## Description

### CROSS-REFERENCE TO RELATED APPLICATION

This application claims priority to and the benefit of Korean Patent Application No. 10-2019-0102121, filed on August 21, 2019, the disclosure of which is incorporated herein by reference in its entirety.

### TECHNICAL FIELD

The present invention relates to a bone marrow harvesting device. More specifically, the present invention relates to a bone marrow harvesting device which, when harvesting bone marrow, can not only easily and stably move the position of a catheter inserted into a bone marrow cavity, but can also harvest the bone marrow in a state where the catheter is stably fixed after the position of the catheter is moved.

### BACKGROUND

Bone marrow is a soft tissue that fills the space between bones, and refers to the tissue that makes blood cells such as red blood cells, white blood cells, and platelets. Such bone marrow has many stem cells which continue to divide and develop to become blood cells. It also has stem cells that make up bones. It is also a very important tissue for the immune system because it produces white blood cells that are responsible for the immune system.

This bone marrow contains two types of stem cells. Among them, hematopoietic stem cells are responsible for the production of blood cells, and continue to divide to become white blood cells, red blood cells, platelets, and the like. Mesenchymal stem cells can differentiate into osteoblasts and chondrocytes that form bones.

Bone marrow may develop malignant tumors or be infected by bacteria, in which case the production of blood cells is reduced. Also, cancer can develop in cells that develop into blood cells, which is called leukemia. These diseases can be diagnosed through bone marrow examination. Bone marrow examination is performed by harvesting red bone marrow using a syringe under local anesthesia.

If the bone marrow does not function normally, a suitable bone marrow of another person may be transplanted. Bone marrow can be harvested using a syringe, as in bone marrow examination.

In this regard, Korean Patent Laid-Open Publication No. 2004-0007321 discloses a bone marrow aspirator, and the bone marrow aspirator described in Korean Patent Laid-Open Publication No. 2004-0007321 includes a handle having proximal and distal surfaces, first and second side surfaces extending between the proximal and distal surfaces, and shape adapted to facilitate grasping thereof; and an elongate penetrating element including a longitudinal axis, a proximal portion coupled to the handle and having a proximal end with an inlet port formed therein, a distal portion having a smooth outer surface and a solid, distal piercing tip, a substantially cylindrical sidewall defining an inner lumen extending from the inlet port to a position proximal to the distal piercing tip, and at least one opening formed in the sidewall and in communication with the inner lumen, the opening positioned proximal to the distal piercing tip, wherein the proximal portion of the elongate penetrating element is offset with respect to a longitudinal axis of the distal portion of the elongate penetrating element such that the handle of the device can be grasped and manipulated to position the distal portion of the elongate penetrating element while simultaneously allowing access to the inlet port in the proximal portion of the elongate penetrating element.

However, the conventional bone marrow harvester including the bone marrow aspirator disclosed in Korean Patent Laid-Open Publication No. 2004-0007321 has a limitation in that it can only harvest a relatively small amount of bone marrow through a small hole positioned proximal to the piercing tip of the elongate penetrating element to be inserted into the bone marrow cavity when harvesting the bone marrow. In other words, there were problems in that since the amount of bone marrow that is sucked into the small hole formed in the penetrating element during the bone marrow harvesting operation is a very small amount of about 1 cc, in order to harvest a larger amount of bone marrow, it is necessary to move the bone marrow harvester to several places and harvest the bone marrow; therefore, since it is not easy to move the bone marrow harvester as described above, the worker has to spend a lot of time and effort in the bone marrow harvesting operation, and the patient is also burdened with a lot of pain.

Therefore, there is an urgent need for improvement in these problems.

The above information disclosed in this Background section is only for enhancement of understanding of the background of the invention and it may therefore contain information that does not form the prior art that is already known to a person of ordinary skill in the art.

### SUMMARY

### Technical Problem

The present invention is directed to providing a bone marrow harvesting device which, when harvesting bone marrow, can easily move the position of a catheter inserted into a bone marrow cavity

In addition, it is directed to providing a bone marrow harvesting device capable of stable operation when the catheter is moved.

Furthermore, it is directed to providing a bone marrow harvesting device capable of harvesting the bone marrow in a state where the catheter is stably fixed after the position of the catheter is moved.

### Technical Solution

The bone marrow harvesting device according to the present invention includes: a hub inserted into a bone marrow cavity; a catheter inserted into the hub and having a syringe detachably connected to one side thereof; and a holder connected to one end of the catheter such that the catheter is movable along the longitudinal direction.

In this case, the hub may be provided with a support bar for fixing the position of the holder, and a support groove into which the holder is inserted and fixed may be formed in the support bar.

In this case, a plurality of support grooves may be formed along the movement direction of the catheter.

In this case, the support grooves may include a first support groove formed on one side of the support bar and a second support groove formed on the other side of the support bar, and the first support groove and the second support groove may be formed at different positions from the bottom surface of the hub.

In this case, the first support groove and the second support groove may be formed to have different distances from each other.

In this case, a mounting portion may be provided so that the support bar is detachable from the hub.

In this case, the mounting portion may include an insert member formed on the support bar and a guide member formed on the hub so that the insert member is inserted and fixed.

In this case, the insert member and the guide member may be respectively formed on one side and the other side of the hub and the support bar, and the insert member and the guide member formed on one side may be formed to have a different height from the insert member and the guide member formed on the other side.

In this case, the holder may include an outer case connected to the hub, and an inner case connected to one end of the catheter, and the inner case may be configured to be disposed inside the outer case, and to be movable along the longitudinal direction of the outer case.

In this case, a first fastening part to which the inner case is fastened may be formed in the outer case, and a second fastening part fastened to the first fastening part may be formed in the inner case.

In this case, a plurality of latching jaws may be formed in the first fastening part along the longitudinal direction of the outer case, and a latching protrusion moving along the latching jaw may be formed in the second fastening part.

In this case, a latching surface having an inclination angle toward the inside of the outer case in the radial direction may be formed on the latching jaw.

In this case, the latching surface may include a first latching surface inclined along a direction in which the inner case is drawn-out, and a second latching surface inclined along a direction in which the inner case is drawn-in.

In this case, the inclination angle of the second latching surface may be formed greater than the inclination angle of the first latching surface.

In this case, the latching protrusion may include a first elastic member that applies an elastic force to the outside in the radial direction of the outer case and a fixing frame that fixes the first elastic member.

In this case, a second elastic member for applying an elastic force in a direction in which the inner case is drawn-in may be provided between the outer case and the inner case.

In this case, it may be configured such that a supporting part for supporting one end of the second elastic member is provided on one side of the outer case, and the latching protrusion supports the other end of the second elastic member.

In this case, the support part may be provided with a coupling member to be attached to or detached from the outer case.

In this case, a first screw member may be formed in the first fastening part along the inner circumferential surface of the outer case, and a second screw member corresponding to the first screw member may be formed in the second fastening part along the outer circumferential surface of the inner case.

In this case, a second elastic member for applying an elastic force in a direction in which the inner case is drawn-in may be provided between the outer case and the inner case.

In this case, it may be configured such that a supporting part for supporting one end of the second elastic member is provided on one side of the outer case, and the second screw member supports the other end of the second elastic member.

In this case, the support part may be provided with a coupling member to be attached to or detached from the outer case.

### Advantageous Effects

In the bone marrow harvesting device of the present invention having the above configuration, the holder is provided to move the position of the catheter put into the bone marrow cavity during bone marrow harvesting, and the hub is provided with the support bar for fixing the position of the holder, so that after the catheter is moved to the correct position, bone marrow harvesting is possible in a stably fixed state.

In addition, the holder is composed of the outer case and the inner case, and since such inner case moves the position of the catheter through the action of moving in the longitudinal direction of the outer case from the inside of the outer case, the position of the catheter is easily moved, thereby improving the convenience of bone marrow harvesting operation.

In addition, since the fastening part is formed in which the outer case and the inner case are fastened each other so that when the position of the catheter is moved, stable operation is possible, the pain felt by the patient during the bone marrow harvesting process can be reduced.

Furthermore, since the positions of the outer case and the inner case are stably fixed using the elastic force of the second elastic member after the position of the catheter is moved, the bone marrow harvesting operation can be stably performed.

### BRIEF DESCRIPTION OF THE DRAWINGS

The above and other aspects, features, and advantages of the present invention will become more apparent to those of ordinary skill in the art by describing embodiments thereof in detail with reference to the accompanying drawings, in which:
FIG. 1 is a cross-sectional view of a bone marrow harvesting device according to an exemplary embodiment of the present invention.
FIGS. 2 and 3 are cross-sectional views of part I-I of FIG. 1, illustrating various embodiments of a support bar.
FIG. 4 is a cross-sectional view of a bone marrow harvesting device according to another exemplary embodiment of the present invention.
FIGS. 5 and 6 are cross-sectional views of part II-II of FIG. 4, illustrating various embodiments of a mounting portion.
FIG. 7 is a cross-sectional view of a bone marrow harvesting device according to yet another exemplary embodiment of the present invention.
FIG. 8 is a cross-sectional view sequentially illustrating a process of harvesting bone marrow using a bone marrow harvesting device according to yet another exemplary embodiment of the present invention.
FIG. 9 is an enlarged view of part A of FIG. 7 and is a view showing a bone marrow harvesting device according to various embodiments.
FIG. 10 is a cross-sectional view showing a bone marrow harvesting device according to yet another exemplary embodiment of the present invention.
FIGS. 11 and 12 are cross-sectional views showing a bone marrow harvesting device according to yet another exemplary embodiment of the present invention.

### DETAILED DESCRIPTION OF THE EMBODIMENTS

Hereinafter, exemplary embodiments of the present invention will be described in detail so that those of ordinary skill in the art can readily implement the present invention with reference to the accompanying drawings. The present invention may be embodied in many different forms and is not limited to the embodiments set forth herein. In the drawings, parts unrelated to the description are omitted for clarity. Throughout the specification, like reference numerals denote like elements.

It is understood that the terms "comprise" or "have" when used in this specification, are intended to specify the presence of stated features, integers, steps, operations, members, components and/or a combination thereof but not preclude the possibility of the presence or addition of one or more other features, integers, steps, operations, members, components, or a combination thereof. In addition, it will be understood that when an element such as a layer, film, region, or substrate is referred to as being "on" another element, it can be "directly on" the other element or intervening elements may also be present. Conversely, it will be understood that when an element such as a layer, film, region, or substrate is referred to as being "below" another element, it can be "directly below" the other element or intervening elements may also be present.

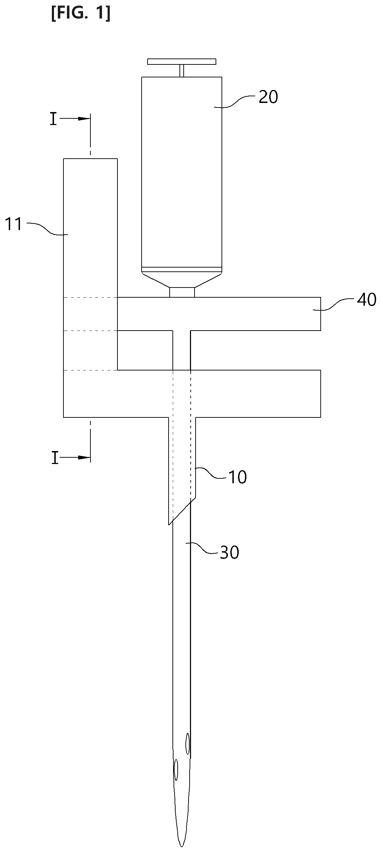

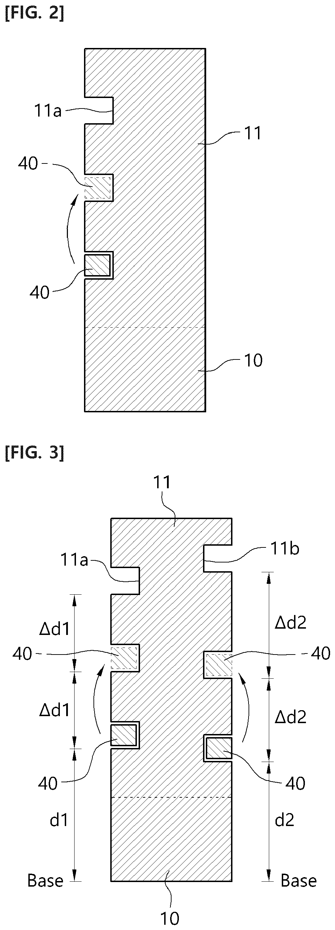

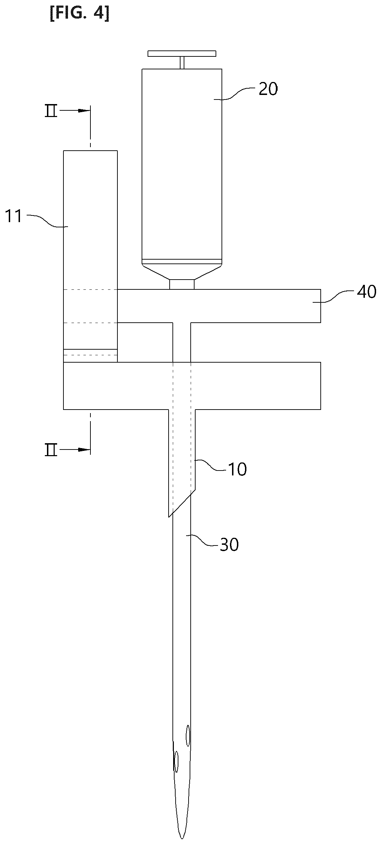

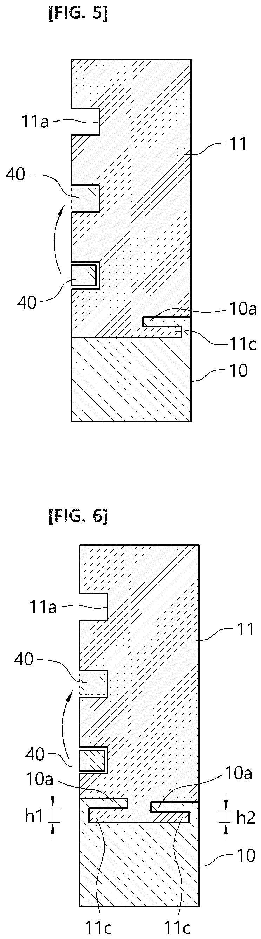

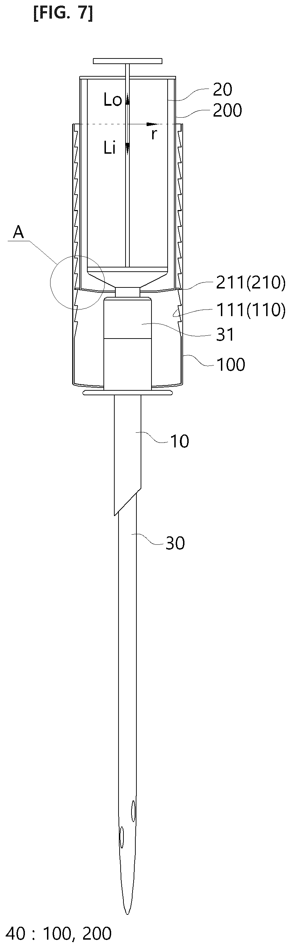

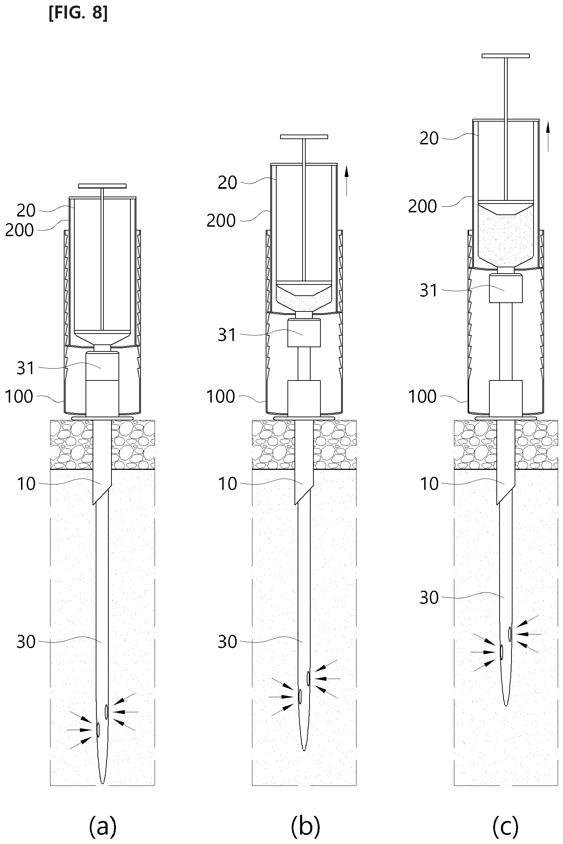

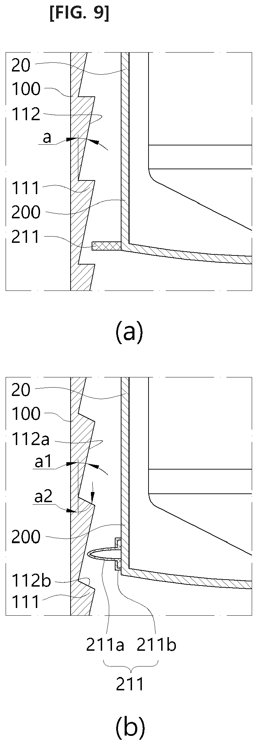

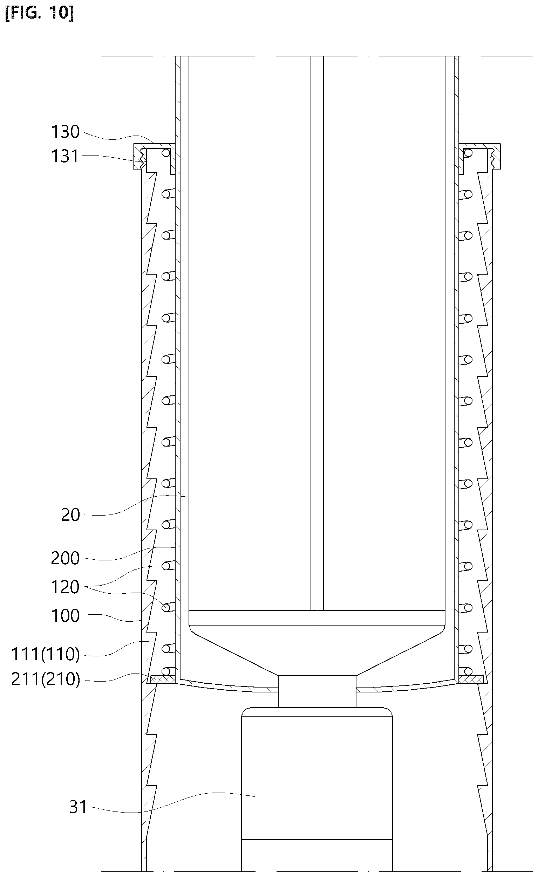

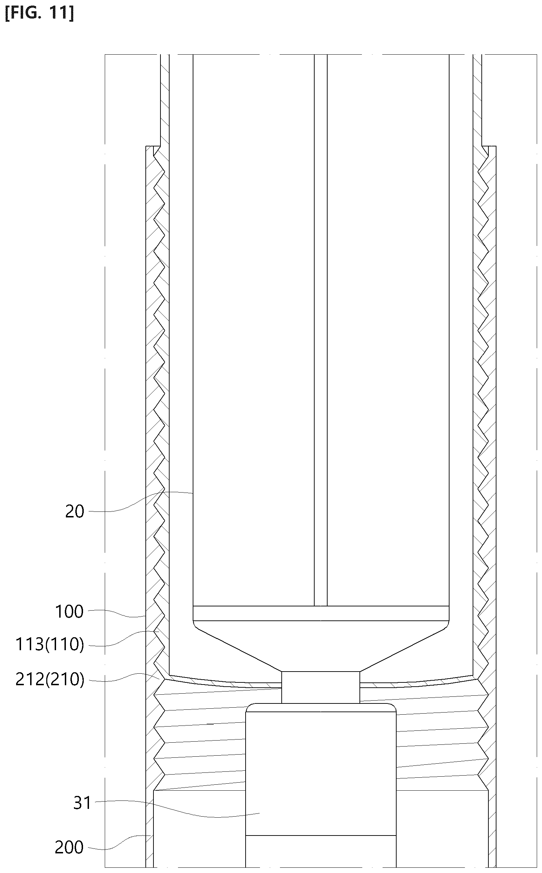

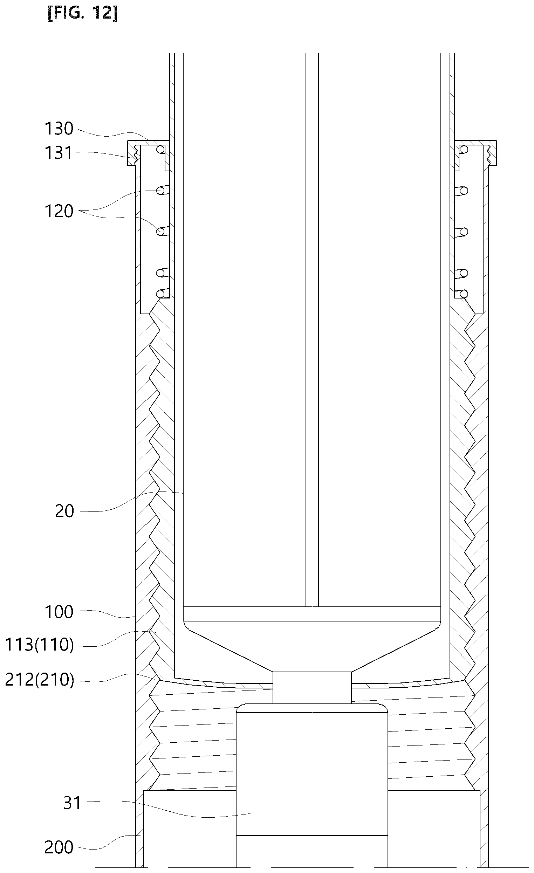

FIG. 1 is a cross-sectional view of a bone marrow harvesting device according to an exemplary embodiment of the present invention, FIGS. 2 and 3 are cross-sectional views of part I-I of FIG. 1, illustrating various embodiments of a support bar, FIG. 4 is a cross-sectional view of a bone marrow harvesting device according to another exemplary embodiment of the present invention, FIGS. 5 and 6 are cross-sectional views of part II-II of FIG. 4, illustrating various embodiments of a mounting portion, FIG. 7 is a cross-sectional view of a bone marrow harvesting device according to yet another exemplary embodiment of the present invention, FIG. 8 is a cross-sectional view sequentially illustrating a process of harvesting bone marrow using a bone marrow harvesting device according to yet another exemplary embodiment of the present invention, FIG. 9 is an enlarged view of part A of FIG. 7 and is a view showing a bone marrow harvesting device according to various embodiments, FIG. 10 is a cross-sectional view showing a bone marrow harvesting device according to yet another exemplary embodiment of the present invention, and FIGS. 11 and 12 are cross-sectional views showing a bone marrow harvesting device according to yet another exemplary embodiment of the present invention.

As shown in FIG. 1, the bone marrow harvesting device according to an embodiment of the present invention includes a hub 10 inserted into a bone marrow cavity; a catheter 30 inserted into the hub 10 and having a syringe 20 detachably connected to one side thereof; and a holder 40 connected to one end of the catheter 30 such that the catheter 30 is movable along the longitudinal direction.

The hub 10 is made of a sturdy material and is first inserted and disposed during the bone marrow harvesting operation, and after the hub 10 is inserted and disposed, the catheter 30 is inserted. The catheter 30 is composed of a flexible material such as polypropylene, it is possible to freely deform the shape during the bone marrow harvesting operation. In addition, a separate guide member (not shown) such as a flexible introducer may be used to guide the catheter 30 to the bone marrow harvesting location. The guide member is inserted into the catheter 30 and passes through the hub 10 to guide the catheter 30 to the bone marrow harvesting location. When the catheter 30 reaches the bone marrow harvesting location, the guide member is removed, and a syringe 20 for bone marrow harvesting is connected to the catheter 30. The catheter 30 may be provided with a head portion 31 for connecting the syringe 20.

In addition, the holder 40 is connected to one end of the catheter 30, and the holder 40 is configured such that the catheter 30 is movable along the longitudinal direction when harvesting bone marrow. That is, in a state in which the hub 10 is inserted, the catheter 30 reaches the bone marrow harvesting location, and when the syringe 20 is connected to the catheter 30, the piston of the syringe 20 is pulled to harvest the bone marrow. When a certain amount of bone marrow is harvested, only the catheter 30 is drawn-out from the hub 10 which is in a state of fixed position. At this time, the syringe 20 is moved together in a state connected to the head portion 31 of the catheter 30. In addition, when a certain amount of bone marrow is harvested even from a newly moved position, the catheter 30 and the syringe 20 are repeatedly drawn-out to harvest bone marrow from a new location.

In this case, as shown in FIG. 2, the above-described hub 10 is provided with a support bar 11 for fixing the position of the holder 40, and support grooves 11a and 11b into which the holder 40 is inserted and fixed may be formed in the support bar 11. That is, the operator proceeds to harvest the bone marrow in a state in which the holder 40 is stably inserted into the support grooves 11a and 11b.

Since a plurality of these support grooves 11a and 11b are formed along the movement direction of the catheter 30, after a certain amount of bone marrow is harvested, the holder 40 is moved to other adjacent support grooves 11a and 11b to be inserted and fixed, and then the bone marrow harvesting operation is continued.

That is, the holder 40 is provided to move the position of the catheter 30 put into the bone marrow cavity during bone marrow harvesting, and the hub 10 is provided with the support bar 11 for fixing the position of the holder 40, so that after the catheter 30 is moved to the correct position, bone marrow harvesting is possible in a stably fixed state.

As shown in FIG. 3, the support grooves 11a and 11b include a first support groove 11a formed on one side of the support bar 11 and a second support groove 11b formed on the other side of the support bar 11, and the first support groove 11a and the second support groove 11b may be formed at different positions from the bottom surface of the hub 10.

That is, all locations from which bone marrow is harvested may be different depending on the patient, and even when the bone marrow harvesting locations are different as described above, the first support groove 11a and the second support groove 11b are formed on one side and the other side of the support bar 11, respectively, so that the holder 40 can be effectively fixed in a state in which the catheter 30 is accurately moved to the corresponding position, and these first support groove 11a and second support groove 11b are formed at different positions from the bottom surface of the hub 10. When the position of the bottom surface of the hub 10 is referred to as the Base, the first support groove 11a is formed at a position spaced apart from the Base by d1, and the second support groove 11b is formed at a position spaced apart from the Base by d2.

In addition, the first support groove 11a and the second support groove 11b may be formed to have different distances from each other. That is, each differently, the distance between the first support grooves 11a is formed by Δd1, and the distance between the second support grooves 11b is formed by Δd2. With this configuration, the operator can harvest bone marrow while moving the catheter 30 by different distances to suit the patient, and furthermore, by using a combination of the first support groove 11a and the second support groove 11b, it is possible to stably harvest bone marrow at various positions.

Alternatively, as shown in FIG. 4, it is also possible to configure the above-described support bar 11 to be detachable from the hub 10. That is, a plurality of support bars 11 in which support grooves 11a and 11b are formed in a state spaced apart by various distances are provided, and the user harvests bone marrow by mounting the support bar 11 suitable for the patient on the hub 10.

To this end, as shown in FIG. 5, mounting portions 10a and 11c may be provided on the support bar 11 and the hub 10, and these mounting portions 10a and 11c may include an insert member 11c formed on the support bar 11 and a guide member 10a formed on the hub 10 so that the insert member 11c is inserted and fixed.

That is, a separation space is formed between the bottom surface of the guide member 10a formed in the hub 10 and the top surface of the hub 10, and the insert member 11c of the support bar 11 is fixed while being inserted into the separation space in a sliding manner.

In this case, as shown in FIG. 5, if the support grooves 11a and 11b are formed on one side of the support bar 11, it is preferable that the aforementioned insert member 11c is formed on the other side of the support bar 11. It is because with this configuration, a state in which the insert member 11c is inserted into the guide member 10a can be stably maintained even in a situation where a force is applied to insert the holder 40 into the support grooves 11a and 11b.

Further, as shown in FIG. 6, it is also possible to form the above-described guide member 10a and the insert member 11c on one side and the other side of the hub 10 and the support bar 11, respectively, and with this configuration, it is possible to stably fix in a situation where the holder 40 is inserted into the support grooves 11a and 11b in any direction.

In addition, it is also possible to form such that the insert member 11c and the guide member 10a formed on one side of the hub 10 and the support bar 11 have a different height from the insert member 11c and the guide member 10a formed on the other side of the hub 10 and the support bar 11. That is, the height of the insert member 11c on one side is formed as h1, and the height of the insert member 11c on the other side is formed as h2. In addition, the height of the separation space formed in the guide member 10a on one side and the guide member 10a on the other side is formed to correspond thereto. With this configuration, when the operator mounts the support bar 11, erroneous assembly of assembling in a state where the direction of one side and the other side is changed can be prevented, and even if the operator does not pay special attention, the support bar 11 can be accurately mounted through these structural features.

As shown in FIG. 7, the holder 40 includes an outer case 100 connected to the hub 10 and fixed in position, and an inner case 200 connected to one end of the catheter 30 and movable along the longitudinal direction (Lo, Li) of the outer case 100. Such inner case 200 is disposed inside the outer case 100, and when the inner case 200 is moved along the longitudinal direction (Lo, Li) of the outer case 100, the catheter 30 connected thereto is also moved.

As such, the holder 40 is provided to move the position of the catheter 30 put into bone marrow cavity, and further, the holder 40 is composed of the outer case 100 and the inner case 200, and since such inner case 200 moves the position of the catheter 30 through the action of moving in the longitudinal direction (Lo, Li) of the outer case 100 from the inside of the outer case 100, the position of the catheter 30 is easily moved, thereby improving the convenience of bone marrow harvesting operation.

That is, as shown in (a) of FIG. 8, in a state in which the hub 10 is inserted, the catheter 30 is moved along the drawing-in direction Li to reach the bone marrow harvesting location, and when the syringe 20 is connected to the catheter 30, the bone marrow is harvested by pulling the piston of the syringe 20. When a certain amount of bone marrow is harvested, as shown in (b) of FIG. 8, only the catheter 30 is moved along the drawing-out direction Lo while the position of the hub 10 is fixed. At this time, the syringe 20 is moved together in a state connected to the head portion 31 of the catheter 30. In addition, when a certain amount of bone marrow is harvested even from a newly moved position, as shown in (c) of FIG. 8, only the catheter 30 and the syringe 20 is moved to harvest bone marrow from a new location.

Such outer case 100 and inner case 200 are configured to allow relative movement along the longitudinal direction of the outer case 100, and to this end, a first fastening part 110 to which the inner case 200 is fastened is formed in the outer case 100, and a second fastening part 210 fastened to the first fastening part 110 is formed in the inner case 200.

As such, since the fastening part is formed in which the outer case and the inner case are fastened to each other, the position of the catheter can be moved stably, so that the pain felt by the patient during the bone marrow harvesting process can be reduced.

In this case, as shown in (a) of FIG. 9, a plurality of latching jaws 111 are formed in the first fastening part 110 along the longitudinal direction (Lo, Li) of the outer case 100, and a latching protrusion 211 moving along the latching jaw 111 is formed in the second fastening part 210. A latching surface 112 having an inclination angle (a) toward the inside of the outer case 100 in the radial direction (r) is formed on the latching jaw 111.

That is, when the inner case 200 is moved in the drawing-out direction Lo, the latching protrusion 211 integrally fixed to the inner case 200 also moves in the same direction, and in this process, the latching protrusion 211 is moved along the latching surface 112 formed on the latching jaw 111. In this case, as described above, since the latching surface 112 is inclined toward the inside in the radial direction (r) of the outer case 100, the latching protrusion 211 is elastically deformed in the process of moving along this latching surface 112, and when reaching a stepped surface 114 on which the latching surface 112 is not formed, the latching protrusion 211 is elastically restored. To this end, it is preferable that the latching protrusion 211 is made of a material capable of elastic deformation.

As described above, through a process in which the latching protrusion 211 is elastically deformed while moving along the latching surface 112 and then, the latching protrusion 211 is elastically restored when reaching the stepped surface 114, the operator can not only move the inner case 200 stably, but also feel through the sense of the hand how much the inner case 200 has moved, thereby improving the convenience of the bone marrow harvesting operation.

Besides, since the latching protrusion 211 is configured to be supported on the stepped surface 114 of the latching jaw 111, the inner case 200 does not move again in the drawing-in direction Li, so bone marrow harvesting is effectively possible.

When the bone marrow harvesting device is initially inserted, a process of inserting the inner case 200 into the outer case 100 and moving the inner case 200 in the drawing-in direction Li is required, and in the process of drawing-in the inner case 200, the latching protrusion 211 may be supported on the stepped surface 114 of the latching jaw 111, but when a force greater than the elastic force of the latching protrusion 211 is applied, the inner case 200 may be effectively drawn-in.

Alternatively, as shown in (b) of FIG. 9, the latching surface 112 may include a first latching surface 112a inclined along a direction Lo in which the inner case 200 is drawn-out and a second latching surface 112b inclined along a direction Li in which the inner case 200 is drawn-in.

That is, it is configured such that in the process in which the inner case 200 is drawn-out, the latching protrusion 211 is elastically deformed while moving along the first latching surface 112a, and then elastically restored when the latching protrusion 211 reaches the second latching surface 112b. At this time, the inclination of the second latching surface 112b may be formed to be smaller than the inclination of the stepped surface 114 of the latching jaw 111 shown in (a) of FIG. 9. That is, assuming that the inclination of the stepped surface 114 of the latching jaw 111 is 90°, the inclination of the second latching surface 112b is formed to be smaller than 90°.

With this configuration, in the process in which the inner case 200 is drawn-out, the latching protrusion 211 repeats elastic deformation and elastic restoration, and through this, the inner case 200 can be stably drawn-out, and even when the bone marrow harvesting device is initially inserted, the latching protrusion 211 is moved while elastically deforming along the second latching surface 112b in the process of drawing-in the inner case 200, thus it is possible to effectively draw-in the inner case 200 even with a small force.

However, it is preferable that the inclination of the second latching surface 112b is formed at an angle that can effectively support the latching protrusion 211 so as not to move again in the drawing-in direction in the process of drawing-out the inner case 200 when harvesting bone marrow.

In addition, it is preferable that the inclination angle a2 of the second latching surface 112b is formed greater than the inclination angle a1 of the first latching surface 112a. With this configuration, when the inner case 200 is drawn-out, the latching protrusion 211 can be effectively elastically deformed along the first latching surface 112a and then elastically restored while reaching the second latching surface 112b.

The latching protrusion 211 may be formed of a single elastic material, as shown in (a) of FIG. 9, or as shown in (b) of FIG. 9, may be configured to include a first elastic member 211a that applies an elastic force to the outside in the radial direction (r) of the outer case 100 and a fixing frame 211b that fixes the first elastic member 211a so as not to be separated.

An elastic member such as a leaf spring may be used as the first elastic member 211a, and with this configuration, through a process in which elastic deformation and elastic restoration of the first elastic member 211a are repeated, the operator can not only move the inner case 200 stably, but also feel more reliably through the sense of the hand how much the inner case 200 has moved, thereby improving the convenience of the bone marrow harvesting operation, and even if elastic deformation and elastic restoration are repeated during the movement of the inner case 100, it is possible to effectively secure the elastic force.

In this case, a second elastic member 120 for applying an elastic force in a direction in which the inner case 200 is drawn-in may be provided between the outer case 100 and the inner case 200.

As shown in FIG. 10, in a state in which the latching protrusion 211 is supported on the stepped surface 114 of the latching jaw 111, the latching protrusion 211 and the latching surface 112 of the latching jaw 111 are spaced apart from each other by a predetermined distance. This is to ensure that the operator can feel the latching protrusion 211 by configuring it so as not to touch the latching surface 112 when elastically restored.

However, since the latching protrusion 211 and the latching surface 112 are spaced apart from each other in this way, the inner case 200 can be additionally drawn-out until the moment when the latching protrusion 211 comes into contact with the latching surface 112, so if the bone marrow is harvested in such a state, the location of bone marrow harvesting may not be accurately maintained.

Therefore, as shown in FIG. 10, when the second elastic member 120 applies an elastic force to the inner case 200 in the drawing-in direction Li of the inner case 200, it is possible to prevent the inner case 200 from being additionally drawn-out after the latching protrusion 211 is elastically restored. That is, since the positions of the outer case 100 and the inner case 200 are stably fixed using the elastic force of the second elastic member 120, the bone marrow harvesting operation can be stably performed.

To this end, it may be configured such that a supporting part 130 for supporting one end of the second elastic member 120 is provided on one side of the outer case 100, and the latching protrusion 211 supports the other end of the second elastic member 120.

In addition, the support part 130 may be provided with a coupling member 131 to be attached to or detached from the outer case 100. With this configuration, it is possible for the operator to adjust the elastic force of the second elastic member 120, such as by installing the second elastic member 120 having a different elastic modulus, or installing a plurality of second elastic members 120, and when the second elastic member 120 is damaged or contaminated, it can be separated and replaced or washed and then reinstalled.

As shown in FIG. 11, a first screw member 113 may be formed in the first fastening part 110 along the inner circumferential surface of the outer case 100, and a second screw member 212 corresponding to the first screw member 113 may be formed in the second fastening part 210 along the outer circumferential surface of the inner case 200.

That is, by rotating the inner case 200, the inner case 200 can be drawn in or out, so that the position of the catheter 30 is easily moved, and since the outer case 100 and the inner case 200 are screwed together, the position can be stably fixed.

In this case, as shown in FIG. 12, a second elastic member 120 for applying an elastic force in a direction in which the inner case 200 is drawn-in may be provided between the outer case 100 and the inner case 200.

As such, when the second elastic member 120 is provided, it is possible to prevent the inner case 100 from being arbitrarily drawn-out. In this case, it is preferable that a stopper (not shown) is provided for preventing the inner case 100 from being arbitrarily drawn-in while rotating by the elastic force of the second elastic member 120.

It may be configured such that a supporting part 130 for supporting one end of the second elastic member 120 is provided on one side of the outer case 100, and the second screw member 212 supports the other end of the second elastic member 120.

In addition, the support part 130 may be provided with a coupling member 131 to be attached to or detached from the outer case 100. With this configuration, it is possible for the operator to adjust the elastic force of the second elastic member 120, such as by installing the second elastic member 120 having a different elastic modulus, or installing a plurality of second elastic members 120, and when the second elastic member 120 is damaged or contaminated, it can be separated and replaced or washed and then reinstalled.

Although exemplary embodiments of the present invention have been described, the spirit of the present invention is not limited to the embodiments set forth herein. Those of ordinary skill in the art who understand the spirit of the present invention may easily propose other embodiments through supplement, change, removal, addition, etc. of elements within the same spirit, but the embodiments will be also within the scope of the present invention.

## Claims

1. A bone marrow harvesting device, comprising:
a hub inserted into a bone marrow cavity;
a catheter inserted into the hub and having a syringe detachably connected to one side thereof; and
a holder connected to one end of the catheter such that the catheter is movable along the longitudinal direction.

2. The bone marrow harvesting device of claim 1, wherein the hub is provided with a support bar for fixing the position of the holder, and a support groove into which the holder is inserted and fixed is formed in the support bar.

3. The bone marrow harvesting device of claim 2, wherein a plurality of support grooves are formed along the movement direction of the catheter.

4. The bone marrow harvesting device of claim 3, wherein the support grooves comprise a first support groove formed on one side of the support bar and a second support groove formed on the other side of the support bar, and the first support groove and the second support groove are formed at different positions from the bottom surface of the hub.

5. The bone marrow harvesting device of claim 4, wherein the first support groove and the second support groove are formed to have different distances from each other.

6. The bone marrow harvesting device of claim 2, wherein a mounting portion is provided so that the support bar is detachable from the hub.

7. The bone marrow harvesting device of claim 6, wherein the mounting portion comprises an insert member formed on the support bar and a guide member formed on the hub so that the insert member is inserted and fixed.

8. The bone marrow harvesting device of claim 7, wherein the insert member and the guide member are respectively formed on one side and the other side of the hub and the support bar, and the insert member and the guide member formed on one side are formed to have a different height from the insert member and the guide member formed on the other side.

9. The bone marrow harvesting device of claim 1, wherein the holder comprises an outer case connected to the hub, and an inner case connected to one end of the catheter, and the inner case is disposed inside the outer case, and is movable along the longitudinal direction of the outer case.

10. The bone marrow harvesting device of claim 9, wherein a first fastening part to which the inner case is fastened is formed in the outer case, and a second fastening part fastened to the first fastening part is formed in the inner case.

11. The bone marrow harvesting device of claim 10, wherein a plurality of latching jaws are formed in the first fastening part along the longitudinal direction of the outer case, and a latching protrusion moving along the latching jaw is formed in the second fastening part.

12. The bone marrow harvesting device of claim 10, wherein a first screw member is formed in the first fastening part along the inner circumferential surface of the outer case, and a second screw member corresponding to the first screw member is formed in the second fastening part along the outer circumferential surface of the inner case.

13. The bone marrow harvesting device of claim 11 or claim 12, wherein a second elastic member for applying an elastic force in a direction in which the inner case is drawn-in is provided between the outer case and the inner case.
